(19) Europäisches Patentamt
European Patent Office
Office européen des brevets

(11) Publication number: **0 506 308 B1**

(12) **EUROPEAN PATENT SPECIFICATION**

(45) Date of publication of patent specification: **10.05.95**

(51) Int. Cl.[6]: **C07C 309/42**, C07C 303/32, C07C 303/06

(21) Application number: **92302417.8**

(22) Date of filing: **20.03.92**

(54) **Method for sulfonating acyloxybenzenes and neutralization of resulting product.**

(30) Priority: **25.03.91 US 674838**

(43) Date of publication of application:
**30.09.92 Bulletin 92/40**

(45) Publication of the grant of the patent:
**10.05.95 Bulletin 95/19**

(84) Designated Contracting States:
**AT BE CH DE DK ES FR GB GR IT LI LU MC NL PT SE**

(56) References cited:
**EP-A- 0 227 194**
**US-A- 4 588 533**
**US-A- 4 692 279**
**US-A- 4 695 412**

(73) Proprietor: **The Clorox Company**
**1221 Broadway**
**Oakland**
**California 94612 (US)**

(72) Inventor: **Ottoboni, Thomas B.**
**2132 Lyon Avenue**
**Belmont, CA 94002 (US)**
Inventor: **Zielske, Alfred G.**
**2282 Via Espada**
**Pleasanton, CA 94566 (US)**
Inventor: **Kuhner, Lisa M.**
**25 Gelding Court**
**Danville, CA 94526 (US)**
Inventor: **Brodbeck, Kevin J.**
**815 Bonde Court**
**Pleasanton, CA 94566 (US)**
Inventor: **Sinisi, Leonardo**
**4913 Cache Peak Drive**
**Antioch, CA 94509 (US)**
Inventor: **Martin, Gregory T.**
**431 Stison No. 5**
**Vallejo, CA 94591 (US)**
Inventor: **Spillett, Cris Tina**
**1800 Camino Verde**
**Walnut Creek, CA 84596 (US)**
Inventor: **Thompson, Suzanne M.**
**5631 Estates Drive**
**Oakland, CA 95618 (US)**
Inventor: **Steichen, Dale S.**
**1432 Sail Court**
**Byron, CA 94514 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

(74) Representative: **Smith, Sydney et al**
**Elkington and Fife**
**Prospect House**
**8 Pembroke Road**
**Sevenoaks, Kent TN13 1XR (GB)**

## Description

The present invention relates to methods for preparing cleaning, bleaching, or fabric treating additives, and more particularly relates to a method for sulfonating an acyloxy substituted benzene ring to obtain high yields of desired sulfonated precursors having cleaning, bleaching, or fabric treating properties when dissolved in aqueous solutions (and in the presence of a peroxygen source for bleaching).

### Background of the Invention

Acyloxybenzenesulfonic acids and their alkali metals and alkaline earth metals salts are acylating agents for amides, mercaptans, hydrogen peroxide, and other compounds containing active hydrogen. They are variously useful in the textile industry as activators for the peroxide bleaching of fabrics and as dyeing assistants in the dyeing of acrylic fibers.

U.S. Patents 4,778,618 and 4,959,187, issued October 18, 1988 and September 15, 1990, respectively, both of inventors Fong et al., describe the uses of certain acyloxybenzenesulfonate compounds in detergent bleaches or as laundry additives having the general formula

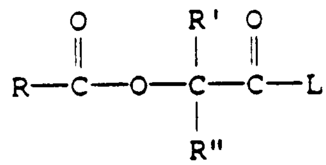

wherein R is $C_{1-20}$ linear or branched alkyl, alkylethoxylated, cycloalkyl, aryl, substituted aryl; R′ and R″ are independently H, $C_{1-20}$ alkyl, aryl, $C_{1-20}$ alkylaryl, substituted aryl, and $NR_3^{a+}$, wherein $R^a$ is $C_{1-30}$ alkyl; and L may be a p-phenyl sulfonate ester. A preferred synthesis of phenol sulfonate esters adaptable for use in preparing these compounds is disclosed in U.S. Patent 4,735,740, issued April 5, 1988, inventor Zielske, where the acid of the desired compound is combined with sodium phenol sulfonate in solvent.

In the Fong et al. description of the preferred acyloxybenzenesulfonate compounds it is most preferred that R′ and R″ are both H, and thus R′-C-R″ is methylene. The alpha hydroxy substituted carbon plus the carbonyl form the glycolate group. When the precursor is combined with a source of hydrogen peroxide, the resulting reaction forms a peracid or a mixture of peracids. The structure and reactivity of these precursor compounds permit higher yields of peracids to be obtained across a broader pH range and temperature than conventional bleach activators.

U.S. patent 4,692,279, issued September 8, 1987, inventor Nussbaum suggests preparing acyloxy benzene sulfonates by direct sulfonation of acyloxybenzene with sulfur trioxide. The sulfonation includes a procedure termed a "temperature controlled digestion step" in which the acyloxy benzene starting material in liquid phase is conducted at a temperature below about 50 °C, preferably below about 30 °C, with sulfur trioxide, and where the initial mole ratio of sulfur trioxide to acyloxybenzene ranges from about 0.9 to 1.1.

U.S. Patent 4,695,412, issued September 22, 1987, inventors Balzer et al. teach that when the phenyl ester to be sulfonated is reacted with sulfur trioxide or chlorosulfonic acid, it is advantageous to maintain a molar ratio of about 1:1 in order to avoid sulfone formation and disulfonation and further by-products. Deviations from the exact molar amount are taught to advantageously amount to no more than 5 mole percent of excess sulfonating agent. Balzer et al. note the acyloxybenzenesulfonic acids are very sensitive to hydrolysis and susceptible to decomposition. A neutralization process is described whereby a 50-50 weight percent aqueous solution of an alkali metal or alkaline earth metal hydroxide, carbonate, or bicarbonate are run simultaneously into water at from 0 °C to 60 °C, preferably from 10 °C to 50 °C, while stirring.

Balzer et al. also suggest effecting sulfonation with the addition of a relatively small amount of an agent which forms a complex with the sulfur trioxide or chlorosulfonic acid. These complexing agents are dioxane, polyalkylene oxides, such as diethylene and dipropylene glycol, whose terminal groups are blocked by alkyl radicals of 1 to 18 carbon atoms, formamide, aliphatic carboxamides of 1 to 10 carbon atoms which are substituted at the amide nitrogen by 1 or 2 alkyl radicals of 1 to 4 carbon atoms, e.g. dimethylformamide, diethylformamide or dibutylformamide, benzamides, 5-membered to 7-membered cyclic amides which are unsubstituted or substituted at the nitrogen by an alkyl radical of 1 to 4 carbon atoms, e.g. N-methylpyrrolid-2-one, N-methylpiperid-2-one or -caprolactam, triazine derivatives, such as melamine, benzoguanamine or acetoguanamine, trialkylamines where alkyl is of 1 to 6 carbon atoms, N,N-$C_1$-$C_4$-dialkylcyclohex-

ylamines, pyridine, triphenylphoshine, amidosulfonic acid, imidazole and boron trifluoride.

U.S. Patent 4,588,533, issued May 13, 1986, inventor Berry also describes a method of preparing acyloxybenzenesulfonic acids and salts and suggests yields may be as high as 70 weight percent and above. Berry exemplifies several sulfonations. In one, sulfur trioxide as a 30% molar excess is reacted with phenyl nonanoate and then neutralized using 30% aqueous sodium hydroxide.

## Summary of the Invention

In one aspect of the present invention, a method for producing additives, such as cleaning, bleaching, or fabric treating additives, is provided that comprises selecting an intermediate having a acyloxy substituted benzene ring, sulfonating the benzene ring of the intermediate, and preferably neutralizing the sulfonated intermediate, to obtain acyloxybenzenesulfonates in high yield. The sulfonating includes two necessary sequential steps: contacting the precursor with a sulfur trioxide source providing at least about a 30% molar excess of sulfur trioxide than is stoichiometrically required; and, irreversibly reacting the complexed and free sulfur trioxide with a quenching agent in the presence of the sulfonated intermediate.

Particularly preferred sulfonated intermediates, or acids and neutralized products, or salts, prepared in accordance with the invention have the structure

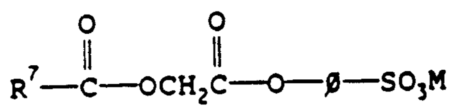

where $R^7$ is a branched or unbranched alkyl with about 5 to 9 carbons and M is an alkali metal, an alkaline earth metal, ammonium, or hydrogen.

In one embodiment of the invention, yields of particularly preferred sulfonated intermediates (that is, where the just illustrated formula has hydrogen as M) on the order of about 80% to 95% in rapid reactions have been obtained from practice of the invention.

In a further embodiment of the invention, the sulfonic acid, or sulfonated intermediate, is then preferably neutralized in a neutralizing step. In the inventive neutralization the neutralized intermediate is in a solid, or precipitated, form and is resistant to degradation such as hydrolysis. The preferred neutralizing step (achieved by either of two embodiments) offers the advantages of maintaining or enhancing overall product stability.

## Brief Description of the Drawings:

Fig. 1 illustrates the sulfonation of a phenyl ester intermediate (here the nonanoyloxyacetic acid embodiment) where the vertical axis is the percentage yield of the sulfonated phenyl ester and the horizontal axis is the initial molar ratio of sulfur trioxide to the phenyl ester intermediate. The curve indicated as representing inventive conditions (b) illustrates practice of the present invention through which about 85% molar yield of the sulfonated intermediate was obtained; and,

Fig. 2 depicts a diagram of a continuous neutralization process for neutralizing the acidic, sulfonated intermediates and formation and drying into noodles.

## Detailed Description of the Preferred Embodiments

In the following discussion the neutralized sulfonated intermediate will sometimes be described by the term "precursor", which means peracid precursor. This term is interchangeable with "bleach activator"and defines a compound (typically an ester) which, when combined with a source of hydrogen peroxide, forms a corresponding peracid molecule. The initial intermediate selected to be sulfonated must have an acyloxy substituted benzene ring and can be prepared by methods known to the art, although the preparation of two such initial intermediates will be described by Example A. The particular intermediate selected for sulfonation will depend, in part, upon the particular application for the ultimate precursor. Sulfonated intermediates (where hydrogen as M is present) and resultant precursors (where M is a counter ion other than hydrogen) resulting from practice of the invention broadly have the Formula I structure:

FORMULA I

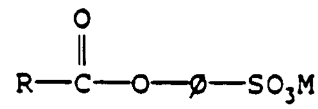

wherein R is $R^1$, $-OR^2$, or

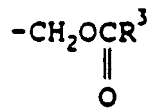

and $R^1$, $R^2$, or $R^3$ is a hydrocarbon radical containing up to about 30 carbon atoms and is selected from alkyl, alkenyl, cycloalkyl, aryl, aralkyl, alkaryl, M is a counter ion such as an alkali metal, an alkaline earth metal, or an ammonium, or is hydrogen.

The Berry patent, U.S. Patent 4,588,533, gives various representative examples for acyloxybenzene compounds suitable as precursors when prepared by the present invention. However, preferred precursors are as described by Fong et al., U.S. Patents 4,778,618 and 4,959,187, and have the structure illustrated by formula II.

FORMULA II

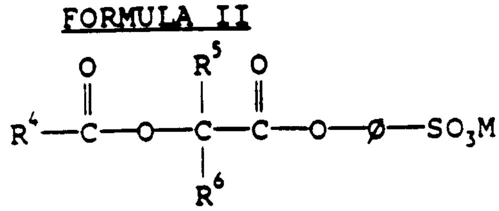

wherein $R^4$ is $C_{1-20}$ linear or branched alkyl, alkylethoxylated, cycloalkyl, aryl, substituted aryl; $R^5$ and $R^6$ are independently H, $C_{1-20}$ alkyl, aryl, $C_{1-20}$ alkylaryl, substituted aryl, and $NR_3{}^{a+}$, wherein $R^a$ is $C_{1-30}$ alkyl; where L of Fong et al. preferably can be a p-phenyl sulfonate. The intermediates of such Formula II precursors have a "M" cation as hydrogen.

Particularly preferred sulfonated precursors are wherein $R^5$ and $R^6$ are both H and $R^4$ is an alkyl. For convenience the particularly preferred sulfonated precursors prepared by the inventive process are illustrated as having the Formula III structure, where $R^7$ is a branched or unbranched alkyl with about 5 to about 12 carbons (and again the intermediates have hydrogen rather than the preferred cations).

FORMULA III

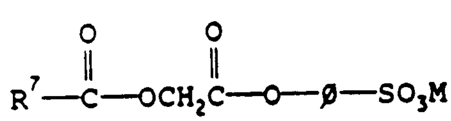

Turning to practice of the inventive method to prepare precursors as just described, we have discovered that during sulfonation of the intermediate's aromatic ring there are competing reactions occurring, particularly at a carbonyl adjacent to the fatty acid carbon chain ($R^4$ of Formula II, $R^7$ of Formula III). This means there is a tendency for coordination with sulfur to occur at that carbonyl carbon as well as sulfonation of the aromatic ring, which leads to the formation of one or more cleavage by-products (when water is added) in addition to the desired sulfonated intermediate. These by-products seriously decrease yields of the desired sulfonated intermediate (and thus ultimately of the desired precursor having the structures of Formulas II and III) when one attempts to sulfonate the intermediate by prior art teachings.

Subsequent to the filing of the application which resulted in U.S. Patent 4,778,618, it was discovered that additional desirable sulfonated precursors, generally named a polyglycolate ester, could be co-produced along with the above precursors, which are called alkanoyloxyglycoylphenyl sulfonates (also

known as alkanoyloxyacetyloxyphenyl sulfonates). This is because the parent carboxylic acid which forms the

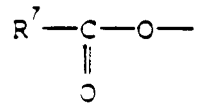

moiety frequently contains some generally low amounts of oligomers, in which the oxyacetyl group is repeated. Thus, in EP-A-0 390 393, a preferred precursor is claimed, having the structure shown below:

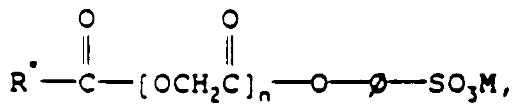

wherein R* is preferably alkyl, M is preferably hydrogen or an alkali metal counterion, and n is >1. These particular precursors are also advantageously produced by sulfonating the appropriate intermediate and neutralizing the sulfonated intermediate thereafter to provide peracid precursors.

The low yield problem posed by prior art sulfonations is illustrated when one sulfonates the phenyl ester of nonanoyloxyacetic acid (abbreviated "NOGB") to obtain the desired sulfonated intermediate, nonanoyloxyacetyloxyphenyl sulfonic acid (abbreviated "NOGPSA"). Thus, sulfur trioxide in methylene chloride solvent (1:1 molar ratio of sulfur trioxide to NOGB) was added to NOGB. The reaction was conducted with mild heating at either 35 °C or 55 °C with the results summarized in Table A.

## TABLE A

(Prior Art Sulfonation)

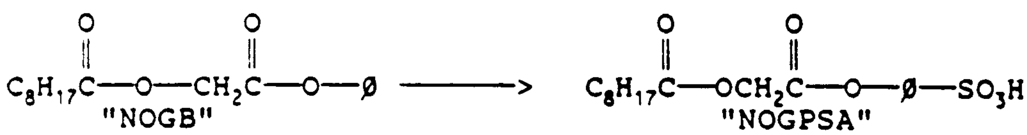

"NOGB" "NOGPSA"

| | | |
|---|---|---|
| 44.3% | a) 4 hours at 35°C | 36.5% |
| 40.8% | b) ½ hour at 55°C | 38.8% |

As can be seen from the summarized reactions in Table A, when the prior art procedure was followed for the intermediate of the Formula III precursor, then less than 40% yields of sulfonated intermediate were obtained. These are unacceptably low yields.

A study of the sulfonation reaction was undertaken that led to an understanding of the competing reactions that occur and cause the unacceptably low yields. Thus, Reaction Scheme I illustrates the sulfonation of a Formula III intermediate (nonanoyloxyacetic acid, phenyl ester) by prior art procedures.

REACTION SCHEME I

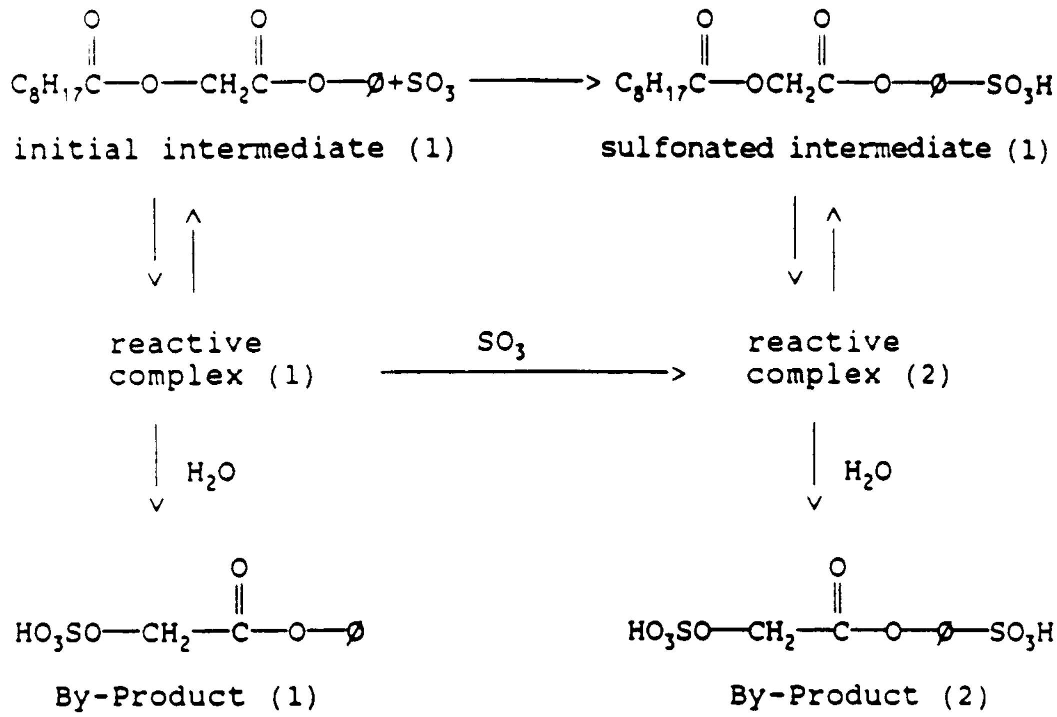


The initial intermediate (1) was reacted with sulfur trioxide in a 1:1 molar ratio in solvent in a range of 0°C to -5°C for three minutes. The reaction was very exothermic, and Table B gives the amounts of different species in the reaction vessel, as determined by high pressure liquid chromatography.

TABLE B

| By-Product 1 | By-Product 2 | Initial Intermediate 1 | Sulfonated Intermediate 1 |
|---|---|---|---|
| 25% | 22% | 26% | 27% |

When the amount of sulfur trioxide was increased to a 100% stoichiometric excess and Reaction Scheme I repeated, then the amount of the initial intermediate (1) was greatly reduced, but without a significant increase in Sulfonated Intermediate (1) as a result. Instead, there was a considerable increase in By-Product (2) formed. This is shown by the data summarized in Table C.

TABLE C

| By-Product 1 | By-Product 2 | Sulfonated Intermediate 1 | Initial Intermediate 1 |
|---|---|---|---|
| 2% | 52% | 44% | 1% |

Despite the Table C data, we have discovered that part of the solution to solving the unacceptable low yields demonstrated by the data of Table B is to use a large excess of sulfur trioxide (or sulfur trioxide from a sulfur trioxide source such as chlorosulfonic acid) where this excess is at least about 30% greater, more preferably is at least about 50% greater, than is stoichiometrically required, most preferably is from greater than about a 50% to about a 100% molar excess (although we have used molar excesses considerably beyond 100% with success).

However, using a substantial excess of sulfur trioxide, by itself, is not sufficient to raise the yield of sulfonated intermediate appreciably, as is shown by the data of Table C. By contrast to practice of the prior

art method that resulted in the data of Table B, and in contrast to simply using sulfur trioxide in a molar excess resulting in the data of Table C, when we used both a substantial molar excess of sulfur trioxide followed by irreversibly reacting "unused" (that is, complexed and free) sulfur trioxide with a quenching agent (as will be hereinafter more specifically described), then significantly increased yields of sulfonated intermediate are obtained, and both by-products, such as illustrated by Reaction Scheme I, are virtually eliminated.

An increased amount of sulfur trioxide, by itself, fails to increase yield of sulfonated intermediate, so also the use of a quenching agent, by itself, does not provide the desired yield increase that one obtains when practicing the present invention.

Accordingly, practice of the present invention requires both (i) contacting the initial intermediate with a substantial molar excess of sulfur trioxide and then (ii) adding a suitable quenching agent that will irreversibly react with complexed and free sulfur trioxide. Quenching agents that do not irreversibly react with sulfur trioxide may give the desired results by a different mechanism. The amount of quenching agent added is preferably about equal to the molar excess of sulfur trioxide, but can widely vary with the molar ratio of quenching agent to excess sulfur trioxide of between about 0.6:1 to very large excesses of quenching agent to excess $SO_3$. However, ratios greater than about 2:1 provide little or no added benefit and can result in significant quenching agent remaining at the end of the reaction that must be removed and recovered.

Turning to Fig. 1, the data points plotted as representing conditions (c) are where no quenching agent was used in sulfonating the NOGB intermediate over a range of initial sulfur trioxide to intermediate molar ratios from 1.1 to 2.5; however, the best molar yield of sulfonated intermediate obtainable even with increased amounts of sulfur trioxide was about 65% when no quenching agent is used. The data points plotted as representing conditions (a) are where quenching agent was used (in an amount about equal to the molar excess of sulfur trioxide), but the molar excess of sulfur trioxide to intermediate was at or below about 30%. Under the conditions (a) a yield of sulfonated intermediate of only about 650 was obtained.

When both a quenching agent and an amount of sulfur trioxide in excess of about 30% were used in sulfonating the initial intermediate, then a molar yield of about 85% sulfonated intermediate was obtained, as is shown by inventive conditions (b). Inventive conditions (b) show that the molar yield rises sharply to a maxima of about 85% yield. The curve falls off after the 85% yield maxima; however, where one increases the amount of quenching agent to greater than the about 1:1 ratio that was used in the conditions of Fig. 1, then the rate of decrease in molar yield can be considerably reduced.

Among the suitable quenching agents for practicing the invention are compounds having at least one aromatic ring (with multiple rings such as in naphthalene or anthracene being possible). The aromatic ring may be unsubstituted, but preferably is substituted with at least one activating group such as $-NH_2$, -OH, $-OCH_3$, $-NHCOCH_3$, $-C_6H_5$, $-CH_3$, or $C_nH_{2n+1}$ (up to about $C_{20}$, branched or unbranched). Toluene and xylene are preferred quenching agents. In some circumstances, it may be desirable to reduce residual toluene. Thus, in such circumstances, it is preferred to first add toluene as the quenching agent, followed by at least one additional, different quenching agent, such as linear alkyl benzene. However, it has unexpectedly been discovered that mixtures of two different quenching agents are also desirable. Especially preferred is a mixture of quenching agents including carboxylic acids and toluene. Preferred activating groups substituted on the aromatic ring are the longer chain alkyls, which provide some detergent properties. Thus, a particularly preferred quenching agent is a linear alkyl benzene that, when irreversibly reacted with unused sulfur trioxide, becomes one of the typical detergent additives for many compositions with which the neutralized sulfonated intermediate (that is, the resultant precursor is usefully formulated. Deactivating, or electron withdrawing groups, should be avoided on the aromatic ring of the quenching agent.

Also among the suitable quenching agents for practicing the invention are carboxylic acids. Particularly preferred carboxylic acids are monoalkyl carboxylic acids where the fatty acid chains are similar to those of the peracid precursors. For example, those monoalkyl carboxylic acids with 5-12 carbons in the monoalkyl group are especially preferred. Thus, when preparing a compound of the invention such as a Formula III compound, then the fatty acid chain of such a carboxylic acid quenching agent may be chosen to be analogous to the $R^7$ of the Formula III compound, unless one does not care that a mixed reaction product (mixed as to $R^7$ chains) will result.

Yet another group of suitable quenching agents are a wide variety of ketones.

Oxides of metals (e.g. magnesium oxide or calcium oxide) are acceptable quenching agents, but tend to be less effective due to slower reactions. Zeolites are acceptable quenching agents, but again are less desirable because they tend to absorb the reaction product. Mixtures of quenching agents hold several advantages. For example, a less expensive quenching agent can be mixed with a more expensive

quenching agent and one quenching agent of the mixture can be assured of being used up completely with the resulting product not incurring undesirable properties. Where more than one quenching agent is used, a premixed combination of the quenching agents can be added or the individual quenching agents can be added sequentially. Where more than one quenching agent is used, then the total amount of quenching agent will typically be about equal to the molar excess of sulfur trioxide.

In some circumstances, where toluene is the selected quenching agent, it may be desirable to reduce the residual toluene. In such circumstances, it is preferred to first add toluene as the initial quenching agent, followed by at least one additional, different quenching agent, such as linear alkyl benzene. However, pre-mixtures of two different quenching agents are also very desirable. Especially preferred mixtures of quenching agents include a combination of carboxylic acids and toluene.

The optimum time for contacting the initial intermediate with the molar excess of sulfur trioxide source (before adding the quenching agent) for a particular intermediate can be determined by monitoring the amount of unsulfonated intermediate and unsulfonated by-product (such as By-Product (1)) during the course of the reaction. For example, with reference to Reaction Scheme I, reaction times of 2 hours at room temperature are typically required to convert essentially all the aryl unsulfonated materials to aryl sulfonated materials when a 50% molar excess of sulfur trioxide is utilized. The reaction times drop drastically with the increasing excesses of sulfur trioxide. Reaction times of 15 minutes are typically sufficient when a 100% molar excess of sulfur trioxide is employed.

The time one wishes to permit the reaction to proceed before adding quenching agent generally depends on the amount of sulfur trioxide present in excess. For example, where the general procedure hereinafter described in Example 1 is modified as to add two equivalents of sulfur trioxide, then the reaction proceeds very quickly and quenching agent can typically be added at about 15 to about 20 minutes.

At least when aromatic quenching agents are used, it has been observed that the quenching reaction appears to occur in two phases. One is a fast phase where a large portion of $SO_3$ is recovered followed by a shorter phase where the remainder of the $SO_3$ is recovered. Also, the second phase of the reaction exhibits a pseudo-zero kinetic order in quenching reagent. Therefore, the amount of quenching agent added need only be enough to consume the excess sulfur trioxide. Moreover, the reaction is largely complete within two hours. Thus, excessive quenching agent and/or reaction time do little to increase the yield of sulfonated product. Increasing the temperature at which the quenching reaction is conducted results in a concomitant increase in the rate of reaction. While it is desirable to maximize the rate of the quenching reaction, the product is thermally unstable. Typically, the temperature of the quenching reaction should be maintained between about 20 °C and 40 °C. Therefore, a balance between maximized yields and minimized reaction times is struck for each particular intermediate. Excessive heating should be avoided, as temperatures even at about 60 °C begin to reduce yield of sulfonated intermediate.

The sulfonation reaction of the invention can be continuous or batch. Where batch, then the reaction medium, or solvent, used preferably has a boiling point higher than the desired reaction temperature, which should not be greater than about 70 °C, and preferably is from about room temperature to about 40 °C. However, reactions may be conducted at temperatures above the boiling point of the solvent, if pressures above atmospheric are used. Examples of solvents which may be used in the process include sulfur dioxide, chlorinated hydrocarbons (especially chlorinated aliphatic hydrocarbons such as methylene chloride, carbon tetrachloride, etc.), aprotic solvents such as triglyme, tetraglyme, 1,2-diethoxyethane, dipolar aprotic solvents such as N,N-dimethylacetamide, tetramethylene sulfone, N-methylpyrrolidinone, and the like. Particularly preferred solvents are methylene chloride or sulfur dioxide.

Rather than performing the inventive process in a solvent, one can use a "falling film" technique, such as is disclosed by U.S. Patent 4,185,030, inventors Brooks et al., issued January 22, 1980. This technique permits continuous manufacturing and is generally preferred for larger scale productions.

Once the sulfonated intermediate is formed and the excess sulfur trioxide is reacted with quenching agent, then the reaction mixture is preferably neutralized in an inventive neutralization process with one or more neutralizing agents to obtain precursors that are useful in applications such as cleaning or bleaching.

In the first embodiment of the neutralization, an alkali solution is used that is concentrated enough to precipitate the reaction product into a solid phase (that is, the amount of water is minimized) yet not caustic enough to hydrolyze ester groups. Sufficient water is present to allow the neutralization reaction to take place in a liquid phase yet which is not in such a great amount to hydrolyze the neutralized intermediate. Thus, the first embodiment neutralizing step includes contacting the sulfonated intermediate with the alkaline neutralizing agent and precipitating substantially all the resulting sulfonated precursor as a solid phase salt, which thus effectively removes the resultant precursor from the neutralizing environment. The formation of the solid phase (precipitated) neutralized precursor prevents the reaction product from decomposing, or hydrolyzing, in the alkaline environment.

In one aspect of the first neutralization embodiment a solution of potassium hydroxide and sodium carbonate may be used. Neutralization in this aqueous solution is advantageous because (1) water in the solution provides a medium for rapid ion exchange ($H^+ \rightarrow K^+/Na^+$) to occur; and (2) the neutralized salt, preferably NOGPS, is only slightly soluble in the solution and precipitates, forming a colloidal dispersion. This colloidal dispersion facilitates product handling characteristics and permits excellent yields. Since the precursor in this form is only slightly soluble in the alkali solution, this minimizes decomposition or hydrolysis. In addition, the combination of potassium hydroxide and sodium carbonate can be used to minimize the amount of water present because one can dissolve up to about 45 wt.% total base in water as opposed to the solubility limit of about 35 wt.% for sodium carbonate.

With this mixture of potassium hydroxide and sodium carbonate (sometimes referred to as a "co-alkali system"), the KOH is useful to minimize the premature precipitation from the concentrated carbonate or caustic solution due the common ion effect. However, other alkali neutralizing agents could be included, such as NaOH, or even, possibly, LiOH. For example, the coalkali system of $KOH/Na_2CO_3$ solution could also be provided as $NaOH/K_2CO_3$. Other alkali metal carbonates, bicarbonates, and sesquicarbonates are possible.

The ratio of alkaline neutralizing agents in the co-alkali system with $KOH:Na_2CO_3$ preferably may be 10:1 to 1:30, more preferably 5:1 to 1:5, and most preferably, 3:1 to 1:3. The KOH may be partly replaced, up to 50%, with NaOH. The amount of alkaline solution added to neutralize the acidic, sulfonated intermediate may be 1-70% active, more preferably 5-60% and most preferably 10-50%. The amount of alkaline neutralizing agent should be provided in a stoichiometric amount versus the amount of sulfonated intermediate being neutralized.

Another particularly preferred neutralizing agent is sodium polyacrylate (e.g., Acusol from Rohm & Haas). The preferred molecular weight of the sodium polyacrylate is 50-100,000, more preferably 100-50,000, and most preferably 1,000-10,000. Sodium polyacrylate serves not only as a neutralizing agent, but also functions as a buffering agent and can act as a binding agent. Excellent yields of neutralized precursor can be obtained from use of sodium polyacrylate as the sole neutralizing agent, but for commercial preparation this is not preferred due to the substantially greater cost of the reagent with respect to salts such as potassium hydroxide and sodium carbonate.

Buffering agents for buffering at about pH 4-5.5 (other than or in addition to the just described sodium polyacrylate) may be included, such as polymaleic anhydride copolymer, polymaleic acid, or polycarboxylic acid salts. Carboxylic acid salts, such as sodium acetate and other inorganic neutralizing agents, may be desirable.

Neutralization is preferably performed as a continuous process in an extruder, although alternative apparatus such as a pug mill may be used. Among the commercially available extruders are those of the dual auger type with a self wiping feature. Either a corotating or a counter-rotating extruder can be used. Neutralization preferably is by one of two neutralization embodiments. In the first neutralization embodiment there is a significant amount of water present, but the amount of water is nevertheless controlled, or minimized and selectively balanced, with the strength of the neutralizing agent. Harshness of neutralizing agent may be modified by buffering, or, in some cases, by dilution.

With reference to Fig. 2, a block diagram of the preferred continuous neutralization process is provided. The alkali solution is blended and reacted with the sulfonated intermediate (here, exemplified by the preferred NOGPSA) with thorough mixing in a vented reaction vessel, to create a neutralized paste or reaction mass ranging in pH from 3-8, but more preferably 4.0-5.5. When the solution contains alkali metal carbonates or bicarbonates, $CO_2$ is generated in the reactor in an amount of 1-7% of the reaction mass. This gaseous $CO_2$ can be removed by mechanical mixing in the reactor and by applying a 2-10 in. Hg vacuum. The resulting neutralized paste is then forced through a 0.25 to 2 mm die to form wet strands. These strands are eventually cut into noodles.

Depending on the formation and amount of water added to the alkali solution, the paste strands will be cut at the die or will require a pre-drying step, to bring water below a 10% level prior to sizing. The predrier must reduce the water content while preserving the shape of the strand (one example of such a drier is a heated belt, or tunnel drier). Possible mechanisms for cutting include rotary knife blades or a turbulent air stream. Once cut, the noodles can be dried to 1-2% water content in a conventional fluid bed or rotary drier.

A dual auger co-rotating extrusion apparatus with venting capability that can be used to conduct the neutralization reactions is commercially available from suppliers such as Warner-Pfleiderer, APV, Teledyne, and others. This type of apparatus can also combine the neutralization and strand formation (noodling) steps. The neutralization reaction generates typically between 15-18 Kcal/mol depending on the type of alkali solution used. NOGPS yield is reduced when the paste temperature exceeds 150 °F. There is a 20%

falloff in yield at 180°F. A significant portion of the heat of reaction is removed upon release (venting) of built-up $CO_2$. Approximately 0.5-0.9% water (5-25% water in the paste) is saturated in the $CO_2$ upon venting. Attempts to remove additional water in a second vent section were unsuccessful at temperatures below 150°F.

Additional formulation ingredients can be added to the extruder at either of two points: (1) the main feed port; or (2) in an added section that would be located between the vent port and the extrusion section. This region would comprise feeding and mixing sections designed to incorporate additional formulation ingredients, e.g., binders, stiffeners, and the like. No neutralizations take place in this region.

Varying the alkali solution's formulation has an effect on the process parameters mentioned above, but does not require significant variation in process equipment configuration. However, there are some particularly preferred modifications in the use of sodium polyacrylate with the first neutralization embodiment or by practicing the second neutralization embodiment.

The neutralizing agent solution of the first neutralizing embodiment is preferably fed into the vented mixing chamber, such as of the earlier mentioned dual auger extruder, where it is blended with the sulfonated intermediate. The amounts of ingredients fed into the chamber are carefully controlled so as to ensure proper pH of the neutralized paste. As earlier noted, other equipment can be utilized, such as (but not limited to) a single auger continuous extruder, a paddle or pug mill mixer, a batch volumetric expansion reactor, a spray dryer, or any other type of liquid atomized reactor.

The resulting neutralized reaction product, or precursor, will typically be in the form of an colloidal suspension, or paste, suitable for extrusion into noodles. A vacuum applied across the extruder can be used to remove carbon dioxide generated in the reaction, some water, or residual solvent.

The vented, dual auger extruder used for many of the examples that follow allows liquids containing the sulfonated intermediate and the neutralizing agent, respectively, to be intimately and discretely brought into contact. The reaction is carefully controlled to prevent exothermic heat of reaction from decomposing the product. The temperature of the reaction chamber is kept below 180°F, more preferably less than 150°F and most preferably less than 120°F. Vacuum evacuation outlets can be used to remove $CO_2$ and water, respectively. As the colloidal suspension or paste is moved through the extruder it could be pushed through a die plate or other sizing means to provide appropriately shaped and sized noodles.

It is especially desirable to combine the finished, neutralized precursors with a suitable binder in order to produce noodles or granules for incorporation into and oxidant-containing detergent, such as are described in EP-A-0 373 743 EP-A-0 507 475.
Thus, the precursor noodles could be combined with a surfactant, water-soluble or water-dispersible polymer, or water-soluble or water-dispersible inorganic material, to obtain bleach activator granules or noodles.

As yet another desirable embodiment of the invention, the steps of neutralizing the acidic intermediate and forming the bleach activator noodle (known as "noodling") could be combined.

One particularly preferred execution of the invention is there linear alkyl benzene (LAB) and toluene are used as the quenching agents in the sulfonation of the intermediates. This is because the surfactant linear alkyl benzene sulfonic acid (HLAS) and the toluene sulfonic acid are formed as a desirable by-products. When the sulfonated intermediate is neutralized to form precursor, the HLAS by-product would also be neutralized, forming the neutralized surfactant, linear alkyl benzene sulfonate, sodium salt (LAS), and the toluene sulfonate, which has been found to improve the integrity of the resulting precursor noodle. From the description in earlier-mentioned EP-A-0 373 743 it is known that LAS is a preferred binding agent for the precursor to form the bleach activator granules and from EP-A-0 507 475 the toluene sulfonate is similarly desirable for inclusion. Thus, from the foregoing, the steps of sulfonation, neutralization and noodling could be combined with significant manufacturing efficiencies and materials cost savings. Accordingly, the combined steps of such a process allows one to obtain final product in a form which is directly utilizable in subsequent processing.

As disclosed in EP-A-0 507 475 added materials may be included, such as stiffeners (calcium or magnesium silicate, fumed or precipitated silica), and co-binders (polyethylene glycol, monoglycerides, hydrocarbon waxes).

In the second embodiment of the neutralization step, water is quite limited during the neutralization. Thus, the overall water level should be controlled to be within about 5 wt.% to about 30 wt.%, more preferably about 5 wt.% to about 15 wt.% of the total weight of the paste or mass. In this second embodiment the amount of water is strictly limited so as to be just sufficient for neutralization to occur at interfaces of the otherwise substantially dry materials.

The use of substantially dry neutralizing agent, such as $Na_2CO_3$ or $NaHCO_3$, or a mixture thereof, such as sesquicarbonate, is preferred but not essential because it reduces the required water content, thus

reducing the amount of drying and increasing ease of processing (due, for example, to reduced foam and easier extrusion). It is preferred, but not essential, in this execution to reduce the substantially dry neutralizing agent particle size such that the average size particle diameter is less than 500 microns, more preferably less than 200 microns, and most preferably less than 100 microns. The reduced particle size increases surface area and thus less water is needed for processing. Desirable sizing can be achieved by milling or grinding if commercial grades are not suitable. The severely limited amount of water dissolves the neutralizing agent substantially only at an interface where the neutralization occurs. However, there must be sufficient water to accommodate mass transfer considerations in order for the neutralization to efficiently proceed. Thus, amounts of water less than about 5 wt.%, such as, for example, at about 3 wt.%, have been found to seriously reduce yield of neutralized precursor.

Either of the above neutralization embodiments could actually be practiced on any synthesis of acidic, sulfonated aryl esters used as bleach activators, and the neutralization aspect of the present invention is believed particularly useful for bleach activators because such compounds are intended to react in the wash water, and thus are highly susceptible to degradation during the neutralization step. For instance, if the synthesis of acidic sulfonated precursors were practiced according to Nussbaum, U.S. 4,692,279, or Balzer et al., U.S. 4,695,412, the yields of acidic, sulfonated intermediate should be quite low. However, in order to recover maximum yields of neutralized product, the neutralization techniques herein could be utilized. Thus, the neutralization steps herein are not limited to the synthesis of the preferred precursors used herein to specifically describe preferred embodiments.

Once the precursor has been formed and neutralized, then it is ready to be used as a bleach additive, in detergent compositions, or in a variety of formulations for other applications. Bleach compositions will typically include a hydrogen peroxide source selected from the alkali metal salts of percarbonate, perborate, persilicate, hydrogen peroxide adducts, and hydrogen peroxide itself. Most preferred are sodium percarbonate, sodium perborate mono- and tetrahydrate. The range of peroxide to neutralized and sulfonated precursor is preferably determined as a molar ratio of peroxide to precursor of from about 0:5 to about 10:1, more preferably about 1:1 to 5:1 and most preferably about 2:1 to 3:1. In addition to various detergents, buffer and filler are typical additional ingredients. Illustrative such components and compositions are further described in Fong et al., U.S. Patents 4,778,618 and 4,959,187.

Aspects and embodiments of the present invention will now be specifically exemplified.

The examples begin with Example A that describes the preferred preparation of two initial intermediates (the phenyl ester of nonanoyloxyacetic acid, sometimes herein abbreviated "NOGB" and the phenyl ester of octanoyloxyacetic acid). Then, aspects of the invention are illustrated by the numbered examples beginning with Example 1.

Example 1 illustrates practice of the present invention with the initial intermediate NOGB (formed as in Example A) under varying conditions where the benzene ring of the intermediate is sulfonated in accordance with the invention, and a sulfonated intermediate reaction product (abbreviated "NOGPSA") is formed in high yield. Example 2 illustrates neutralization of the sulfonated intermediate from the acid form to a salt, or precursor, form (the particular embodiment being designated in abbreviated form "NOGPS"). Example 3A is analogous to that described for Example 1, but with another intermediate. Example 3A illustrates practice of the present invention where the quenching agent is a carboxylic acid. Example 3C illustrates practice of the present invention where a ketone is the quenching agent. Examples 3D and 3E illustrate practice of the present invention where a mixture of quenching agents is used.

Beginning with Example 4, the two neutralization embodiments are particularly illustrated. The typical end product from the neutralization was nonanoyloxyglycoylphenyl sulfonate, sodium salt (NOGPS). Example 4A illustrates some surprising results in which a relatively mild neutralizing agent in relatively low amounts, on one hand, gave relatively low yields of neutralized products while, on the other hand, a harsh alkaline agent in relatively concentrated form gave about the same relatively low yields of neutralized product. Beginning with Example 4B we show how these two extremes, both leading to relatively low yield of neutralized product, can be avoided through practice of the first embodiment neutralizing step. Examples 5 and 6 likewise exemplify practice of the first embodiment neutralizing step, but with different types of neutralizing agent solutions. Example 6 also shows the effects of buffering. Examples 7, 8, and 9 illustrate aspects of the second embodiment neutralization step.

EXAMPLE A

Preparation of Octanoyloxyacetic Acid, Phenyl Ester

To a slurry of 6.9 g of powdered potassium carbonate in 50 ml of dimethylformamide was added 4.4 ml of nonanoic acid followed by 4.25 g of distilled chloracetoxybenzene (prepared according to the procedure given in the *Journal of Organic Chemistry*, Vol. 24, 1523-1526). The resulting mixture was stirred for 25 hrs. at 25 °C and then poured over 250 ml of ice water and the product extracted with ethyl acetate (3 X 125 ml). The combined extracts were washed with water (3 X 200 ml), dried over magnesium sulfate, and stripped to give 6.22 g of yellow oil. The crude product was purified by flash column chromatography eluting with 10% ethyl acetate in hexane to give 4.6 of colorless oil. NMR ($CD_2Cl_2$) 0.9 (t, 3H, $CH_3$), 1.3 (m, 12H), 1.7 (m, 2H), 5.83 (s, 2H, $CO_2CH_2CO_2$), 7.1 (m, 2H), 7.25 (m, 1H), 7.4 (m, 2H).

Preparation of Nonanoyloxyacetic Acid, Phenyl Ester

To a stirred flask containing 258 g of 99% crude chloroacetoxybenzene (prepared by a modification of the procedure given in the *Journal of Organic Chemistry*, Vol. 24, 1523-1526, in which an excess of chloroacetyl chloride was employed and the excess chloroacetyl chloride removed from the produce by sparging with dry nitrogen), 2.7 l acetonitrile, 3.3 g of tetramethylammonium chloride, and 159 g of powdered sodium carbonate was added 60 ml of a solution prepared from 281 ml of 98% nonanoic acid in 300 ml of acetonitrile. The mixture was then heated to 75 °C over 70 min. During this time a further 320 ml of the nonanoic acid solution was added dropwise. The remaining nonanoic acid solution was added during the first 20 min. at 75 °C. After the nonanoic acid addition was complete, the mixture was held at 75 °C for an additional 2.5 hrs. The solvent was then removed under reduced pressure and the residue washed successively with 2.5:1 and 1:1 portions of water. The cloudy organic layer was dried over magnesium sulfate and filtered to give 329.3 g of amber oil.

The product was analyzed by gas chromatography using a HP-I capillary column with FID detector and using the following temperature program: 5 min. at 100 °C, following by a 20 °C/min. temperature increase until the temperature reached 200 °C. The following components were noted (GC area %): 0.84% phenol, 2.62% 2-methyloctanoyloxyacetic acid, phenyl ester, 91.32% nonanoyloxyacetic acid, phenyl ester, and 3.48% nonanoyloxyacetoxyacetic acid, phenyl ester.

Alternatively, preferred procedures for producing these phenyl esters are provided by the teachings of co-pending applications of Dumas entitled "Improved Process for Preparing Phenyl Chloroacetate", filed concurrently herewith, and "Improved Process for Preparing Phenyl Esters of Substituted Acids," filed concurrently herewith.

EXAMPLE 1

The general procedure followed was to react 1.5 equivalents of $SO_3$ (based upon NOGB) in solvent for 2 to 2.5 hrs. at 20 °C. One equivalent of a quenching agent based upon the excess of $SO_3$ was then added, and the reaction mixture permitted to stand at 20 °C or gently heated (40 °C or 60 °C) for either 4 hrs. or 20 hrs. Table 1 summarizes eight representative such sulfonations in accordance with the invention, followed by the specific details of run (5).

TABLE 1

| Run # | Solvent | Quenching Agent | Time & Temp. with Quenching | Yield of Sulfonated Intermediate (mole %) |
|---|---|---|---|---|
| 1 | methylene chloride | toluene | 4 hr., 40 °C | 88 |
| 2 | methylene chloride | toluene | 20 hr., 40 °C | 89 |
| 3 | methylene chloride | LAB | 4 hr., 40 °C | 86 |
| 4 | methylene chloride | LAB | 20 hr., 40 °C | 87 |
| 5 | sulfur dioxide | LAB | 2 hr., 20 °C | 90 |
| 6 | sulfur dioxide | LAB | 2 hr., 20 °C | 88 |
| 7 | sulfur dioxide | LAB | 2 hr., 40 °C | 84 |
| 8 | sulfur dioxide | LAB | 2 hr., 60 °C | 77 |

Run #5 was a particularly good run in terms of time, temperature, and yield. This run was conducted specifically as follows. To a well stirred solution of 2.92 g of nonanoyloxyacetic acid phenyl ester ("NOGB") in approximately 7 mL of liquid sulfur dioxide maintained at -10 °C was slowly added 0.6 mL (1.2 g, 1.5 equivalents) of neat sulfur trioxide via syringe. The resulting solution was allowed to stir at -10 °C for 2.5 hours and then LAB (1.5 mL, 1.3 g 1.0 equivalent to excess $SO_3$) was added. The reaction flask was allowed to warm to room temperature, whereby all the sulfur dioxide solvent evaporated. The resulting reaction mixture was allowed to stir at room temperature for 2 hours. A sample was withdrawn, added to aqueous buffer to stop the reaction, and analyzed by HPLC.

EXAMPLE 2

To a well stirred solution of 29.2 gm of nonanoyloxyacetic acid phenyl ester, NOGB, (85% pure) in 65 mL of methylene chloride maintained at 0 °C was slowly added 12 gm of sulfur trioxide (1.5 equiv.). The resulting solution was allowed to warm to room temperature. After this mixture had stirred for 2.5 hr., 13.5 mL of tridecylbenzene was rapidly introduced into the solution. This mixture was stirred at 40 °C for 4 hr., after which time a sample of the reaction mixture was withdrawn from the vessel. Analysis of this solution indicated that the reaction vessel contained 26.2 gm of nonanoyloxyacetic acid phenylsulfonic acid ester (31.6 gm theoretical), corresponding to an 83% yield, (93% accounting for unreacted NOGB). The solvents were removed under reduced pressure at 50 °C to give 54 gm of an amber oil that was immediately neutralized. Neutralization can be accomplished in accordance with the reaction procedures depicted in Examples 4-10 below.

EXAMPLE 3A

Another intermediate was sulfonated in accordance with the invention to result in sulfonated intermediate having the Formula I structure where R is $C_9H_{19}$. The procedure was analogous to that described by Example 1. The intermediate was in a molar ratio with respect to sulfur trioxide of 1:1.5. The first step was conducted at a temperature of 23 °C for 2 hrs. A molar excess of toluene as quenching agent (10:1) was then added and the reaction was quenched at a temperature of 23 °C for 2 hr. The resultant sulfonated intermediate was obtained in a yield of 92.6%.

Similarly, sul fonated intermediates were prepared in accordance with the invention of the Formula II structure where $R^4$ was $C_5H_{11}$, $R^5$ was $CH_3$ and $R^6$ was $C_2H_5$.

EXAMPLE 3B

A Parr bench-top mini pressure reactor (300 ml capacity, stainless steel) was utilized when sulfur dioxide was used as the reaction solvent. The reactor head was equipped with magnetic stirrer, thermocouple, gas release valve, gas inlet valve, and a ball joint with rubber septum. The liquid NOGB was added to the reactor cylinder, the cylinder flushed with nitrogen, and then the pressure reactor head was fastened onto the cylinder. The reactor was cooled to minus 25 °C by means of an external dry-ice/acetone bath. The sulfur dioxide gas was condensed into the pressure reactor from a lecture bottle (on a pan balance) connected to the gas inlet valve on the reactor head via a rubber hose. Sufficient sulfur dioxide was added so that the resulting solution was 1M in NOGB. Then neat sulfur trioxide in a syringe (1.6 equivalents based on NOGB) was added to the reactor contents by means of the ball joint equipped with a rubber septum. The reactor was warmed to 20 °C with an external water bath and kept at this temperature, with stirring, for two hours.

The pressure reactor was then cooled, by an external dry-ice/acetone bath, to minus 25 °C. Nonanoic acid (one equivalent based upon excess sulfur trioxide used), in a syringe, was added to the reaction contents by means of the ball joint equipped with rubber septum. The reactor was heated to 40 °C with an external oil bath and kept at this temperature, with stirring, for two hours. The sulfur dioxide solvent was then allowed to escape from the reactor and bubble through cold, aqueous sodium hydroxide by means of rubber tubing connected to the gas release valve of the pressure reactor head. The reactor head was then removed from the reactor cylinder and the viscous, reddish liquid product recovered. The product was analyzed by high pressure liquid chromatography (HPLC) and found to contain 96.2% of the desired sulfonated intermediate.

EXAMPLE 3C

In a manner analogous to Example 3B, but where methyl amyl ketone (one equivalent based upon excess sulfur trioxide used) was the quenching agent, the desired sulfonated intermediate was obtained in a yield of 91.9%.

EXAMPLE 3D

Two different quenching agents were used sequentially in preparing the desired sulfonated intermediate. The sulfonation procedure outlined in Example 3B was followed. The quenching step began by cooling the reactor with an external dry-ice/acetone bath to minus 25°C. Toluene (0.6 equivalents based upon excess sulfur trioxide) in a syringe was added via the ball joint with rubber septum. The reactor was warmed to 40°C by means of an external oil bath and the reaction allowed to stir for one hour. The reactor was again cooled with the dry-ice/acetone bath, nonanoic acid (0.4 equivalents based upon excess sulfur trioxide) in a syringe was added and the reactor heated to 40°C with the oil bath. The reaction was stirred for two more hours at 40°C and then worked up and analyzed as described in Example 3B. The yield of desired sulfonated product was 96.5%.

Alternatively, the toluene and nonanoic acid can be premixed and then added to the reaction. This mixture was heated at 40°C, with stirring, for two hours and worked up and analyzed as described above. The yield of desired sulfonated product was 94.3%.

EXAMPLE 3E

In a manner analogous to Example 3D, toluene (0.6 equivalents based upon excess sulfur trioxide) and linear alkylbenzene, LAB, (0.2 equivalents based upon excess sulfur trioxide) were used as quenching agents (added sequentially). When worked up and analyzed as described above, the yield of desired sulfonated product was 88.6%.

EXAMPLE 4A

In one experiment 3 wt.% $NaHCO_3$ was used to neutralize NOGPSA (that had been toluene quenched), but the resulting yields of NOGPS were relatively low (61%). Thus the mild bicarbonate base in a weak concentration was found to give a low yield of neutralized precursor. These results were surprising because one would imagine that a product quite sensitive to degradation such as the sulfonated intermediate being neutralized here would be best neutralized with a mildwith base.

By using concentrated solutions of a harsher base (30 wt.% $Na_2CO_3$), yields improved to 95-100%. These results would tend to lead one to expect that an even harsher, or stronger, neutralizing agent might be better.

However, if a very harsh alkaline neutralizing system, e.g., 50% NaOH solution is used, the precipitated NOGPS end product apparently is attacked by the strong alkali, reducing yields to 50-60%.

It is speculated, or theorized, that hydrolysis of the product may be occurring in the milder $NaHCO_3$ solution due to the greater amount of water present. If the relatively strong $Na_2CO_3$ solution is used, the NOGPS formed precipitates out of solution and thus appears less capable of reacting with the basic neutralizing system. Through these observations and the theory adduced, the concentrations and strong base effects were exploited selectively to maximize product yield, as described in Examples 4B, 5, and 6.

EXAMPLE 4B

A combination of soda ash (either $Na_2CO_3$ or $K_2CO_3$) with caustic (NaOH) or potash (KOH) was used. by using this co-alkali system, water content can be minimized and the yields of product maximized since the alkaline environment is not as harsh and does not attack the precipitated NOGPS end product significantly. By neutralizing NOGPSA (that had been toluene quenched) with a 35 wt.% $KOH/Na_2CO_3$ solution, in a weight ratio of 3:7, yields of 95-100% were achieved. The moisture content of the resulting end product was 21 wt.% water, as compared to a content of 28 wt.% when $Na_2CO_3$ was used as the sole alkaline neutralizing agent.

EXAMPLE 5

In a manner substantially similar to that of Example 4B the acidic, sulfonated intermediate, NOGPSA, which may be produced in accordance with Examples 1-3 above, was neutralized by using another co-alkali solution. In this example, a co-alkali system of 10.5% KOH, 24.5% $Na_2CO_3$, and 65% $H_2O$ was used. The neutralized end product was extruded as a paste or formed into noodles and dried.

EXAMPLE 6

In this example sodium polyacrylate (Acusol LMW-45N, Rohm & Haas) was used as a buffering agent to improve yields. The improved results were surprisingly noteworthy. When a strong alkaline neutralizing agent, 50% NaOH solution was used, yields of 50-60% NOGPS were obtained. However, by adding sodium polyacrylate (at 20 wt.% of total), the yields improved to 80%. Thus, ameliorating the harsh alkali can improve product yields significantly.

This was performed with a solution of 86.2-100.0% sodium polyacrylate (Acusol LMW-45N) (0.1-1.0 equivalents), and 0-13.8% NaOH (50% solution) used as the neutralizing agent. The polyacrylate can function also as a binding agent for the NOGPS noodle.

In this example, there was no carbonate or bicarbonate in the alkali, so a smaller vent section can be used. There is still a need to vent gases because although $CO_2$ is not generated, gases dissolved in the NOGPSA and liquid alkali can be released upon formation of the neutralized product, NOGPS.

EXAMPLE 7

In this example, a stoichiometric amount, or a slight excess of solid $Na_2CO_3$ was used as the neutralizing agent, followed by only enough water (5-15%) to moisten the mass. The actual amount of water within the range depends on the degree of mixing.

If this solid $Na_2CO_3$ is used as the alkali, the feed section must be modified so that the solids and liquids do not prematurely mix and react prior to entering the extruder. Solid $Na_2CO_3$ was fed first into the main hopper and then followed with the addition of separate streams of liquids (the acidic, sulfonated intermediate, and water) through a separate feed port, slightly downstream of the main hopper. However, total water from all sources was only about 8%. Feed screws which contact the extruder walls can prevent material (the reaction mass) from building up on the wall. Material buildup can cause the eventual binding of the auger, impairing mixing. Yield of neutralized product was 95%.

EXAMPLE 8A

Dry mixing (sometimes "dry neutralization") was attempted in a batch procedure. A liquid stream of acidic, sulfonated precursor (from a LAB quench) was sprayed onto a bed of dry alkali such as $Na_2CO_3$ with water being about 1% of total. While an alternate neutralization, this technique is not as preferred as that described in Examples 7 and 8B because it requires a long induction period for the reaction to occur due to the fact there is not sufficient water as a medium in which rapid ion exchange or mass transfer can take place. Moreover, yields are difficult to quantify because of the presence of unreacted material presenting an unfavorable environment for the stability of the end product. One day yield measurements were only 40-60%.

EXAMPLE 8B

In a manner similar to Example 7 but in contrast to Example 8A, the dry neutralization can be unexpectedly enhanced by adding sufficient water to moisten the reaction mass. First, dry $Na_2CO_3$ and NOGPSA (from a toluene quench) in a 1:5.2 weight ratio were admixed (in a batch procedure), then water was added to moisten in an amount of about 10% total. It is believed that the water acts as a medium for ion ($H^+$ and $Na^+$) exchange. A product yield of 96% was achieved.

EXAMPLE 9

In this example, a slightly "wetter" neutralizing agent was utilized in a batch procedure. A $Na_2CO_3$ slurry, or a co-alkali slurry of $Na_2CO_3$/KOH, was used to contact an acidic, sulfonated intermediate, NOGPSA. Water was 13% of the total reaction mass. Product yields similar to Example 8B were achieved.

Thus the order of addition of water and base may not be significant, but total amount of moisture and the type of alkali are both important selectively to achieve high yields.

EXAMPLE 10

The acidic, sulfonated intermediate (here decanoylphenolsulfonic acid) produced by practice of the inventive quenching aspect (here LAB quench) was neutralized in a batch procedure with a stoichiometric amount of a 25 wt.% $Na_2CO_3$ solution. The resultant precursor was sodium decanoylphenolsulfonate in a yield of 86%.

EXAMPLE 11

Acidic, sulfonated intermediate (NOGPSA) was entirely neutralized with sodium polyacrylate (Acusol LMW-45N) in a weight ratio of 1.3:1 to give the precursor in a yield of 99%. This was a batch neutralization, and shows the superior yields that can be achieved with the sodium polyacrylate, which functions as neutralizing and a buffering agent.

EXAMPLE 12

In a batch neutralization the NOGPSA was first mixed with bicarbonate and then water was added to give an overall water content of 10% in the paste. The NOGPSA was prepared from a mixed quench. The mixture of NOGPSA and sodium bicarbonate was where the sodium bicarbonate was in a slight molar excess. The precursor NOGPS was obtained in a 98% yield.

EXAMPLE 13

Sulfonated intermediate NOGPSA (from a mixed quench) was neutralized with a stoichiometric amount of a slurry made from mixing 0.36 equivalents of Acusol LMW-45N and 0.64 equivalents of milled soda ash. The resultant neutralized paste had a water content of 15% with the water source being mainly the Acusol. A 96% yield of NOGpS precursor was obtained.

It is to be understood that while the invention has been described above in conjunction with preferred specific embodiments, the description and examples are intended to illustrate and not limit the scope of the invention, which is defined by the scope of the appended claims.

## Claims

**1.** A method for producing cleaning, bleaching, or fabric treating additives comprising:
selecting an initial intermediate having an acyloxy substituted benzene ring;
sulfonating the benzene ring of the initial intermediate, the sulfonating including (a) contacting the initial intermediate with a sulfur trioxide source, the sulfur trioxide source providing at least about a 30% molar excess of sulfur trioxide than is stoichiometrically required for sulfonating the benzene ring to form a sulfonated intermediate and then (b) irreversibly reacting excess sulfur trioxide with at least one quenching agent in the presence of the sulfonated intermediate.

**2.** The method as in claim 1 wherein the total quenching agent is added in an amount of about at least 0.6:1 with respect to the molar excess of sulfur trioxide.

**3.** The method as in claim 1 further comprising:
neutralizing the sulfonated intermediate to form a peracid precursor.

**4.** The method as in claim 1 wherein the at least one quenching agent has at least one aromatic ring, or is a carboxylic acid, or a ketone.

**5.** The method as in claim 4 wherein the aromatic ring is unsubstituted or is substituted with at least one activating group.

**6.** The method as in claim 5 "herein the activating group is selected from the group consisting of $-NH_2$, -OH, $-OCH_3$, $-NHCOCH_3$, $-C_6H_5$, $-CH_3$, or $C_nH_{2n+1}$, there n is an integer up to about 20.

7. The method as in claim 1 wherein the at least one quenching agent is toluene, a linear alkyl benzene, xylene, a carboxylic acid, or a ketone, and when more than one quenching agent, then said quenching agents are added together or sequentially.

8. The method as in claim 1 wherein the initial intermediate has the structure

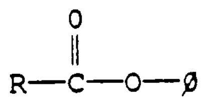

R is $R^1$, $-OR^2$, or

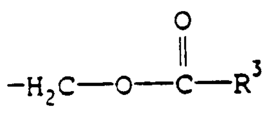

where $R^1$, $R^2$, or $R^3$ is a hydrocarbon radical containing up to about 30 carbon atoms and is selected from the group consisting of alkyl, alkenyl, cycloalkyl, aryl, aralkyl, and alkaryl.

9. The method as in claim 1 wherein the initial intermediate has the structure

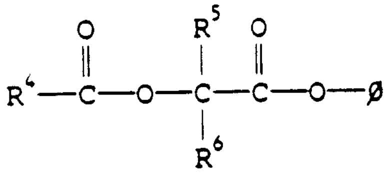

wherein $R^4$ is $C_{1-20}$ linear or branched alkyl, alkylethoxylated, cycloalkyl, aryl, substituted aryl; $R^5$ and $R^6$ are independently H, $C_{1-20}$ alkyl, aryl, $C_{1-20}$ alkylaryl, substituted aryl, and $NR_3{}^{a+}$, wherein $R^a$ is $C_{1-30}$ alkyl.

10. The method as in claim 1 wherein the initial intermediate has the structure

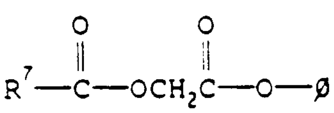

and $R^7$ is a branched or unbranched alkyl with about 5 to about 9 carbons.

11. The method as in claim 9 or 10 wherein the at least one quenching agent added is xylene, a carboxylic acid, a ketone, toluene, or a linear alkyl benzene.

12. The method as in claim 9 or 10 wherein the total quenching agent is present in an amount about equal to the molar excess of sulfur trioxide.

13. The method as in claim 1 wherein the contacting of step (a) includes monitoring either an amount of a first reaction by-product or an amount of sulfonated intermediate being formed before the complexing of step (b), and adding at least one quenching agent when the amount being monitored reaches a selected value.

14. The method as in claim 3 wherein the irreversible reaction of step (b) includes monitoring either an amount of a second reaction by-product or an amount of sulfonated intermediate formed before adding quenching agent, and neutralizing when the amount being monitored reaches a selected value.

**15.** The method as in claim 3 wherein the neutralizing includes contact ing the sulfonated intermediate with a neutralizing agent to neutralize the sulfonic acid and to form a peracid precursor, and precipitating substantially all the peracid precursor as a solid phase salt.

**16.** The method of claim 15 wherein said neutralizing agent is an alkali metal carbonate, bicarbonate, or a mixture thereof.

**17.** The method of claim 15 wherein said neutralizing agent comprises a mixture of potassium or sodium hydroxide and an alkali metal carbonate.

**18.** The method of claim 15 wherein said neutralizing agent comprises a mixture of alkali metal polyacrylate and alkali metal carbonate.

**19.** The method of claim 15 wherein said neutralizing agent comprises a mixture of alkali metal polyacrylate, alkali metal hydroxide and/or alkali metal carbonate.

**20.** The method as in claim 15 wherein the contacting occurs in the presence of a selected amount of water.

**21.** The method of claim 20 wherein the quenching agent is toluene, carboxylic acid, or linear alkyl benzene, a mixture of toluene and linear alkyl benzene, or a mixture of toluene and carboxylic acid, and the resulting sulfonated intermediate and by-product are both neutralized.

**22.** A method for preparing peracid precursors in high yield by neutralizing sulfonated aryl esters, comprising:

providing a neutralizing formulation with components thereof consisting essentially of an intermediate compound to be neutralized, a neutralizing agent, and a liquid mass transfer agent, the intermediate compound having an aryl ester moiety with a sulfonic acid substituent on the aromatic ring, the neutralizing agent providing a source of cations exchangeable with the hydrogen ions of the sulfonic acid substituent for neutralization thereof, and the liquid mass transfer agent permitting exchange of said cations and hydrogen ions in a liquid medium, such exchange forming a peracid precursor as a neutralized intermediate, the neutralizing agent and the liquid mass transfer agent of the formulation being selected to permit formation of neutralized intermediate as a solid within the neutralizing formulation when the components thereof are placed into intimate contact; and,

intimately contacting said formulation components to form neutralized intermediate as a solid within the neutralizing formulation during the contacting, the solid neutralized intermediate being substantially resistant to hydrolysis by said neutralizing formulation.

**23.** The method as in claim 22 wherein the neutralizing agent is selected from the group consisting of alkali metal hydroxide and alkali metal polyacrylate, alkali metal carbonate, alkali metal bicarbonate, and mixtures thereof.

**24.** The method as in claim 22 or 23 wherein the liquid mass transfer agent is water and the water total is maintained at between about 5 wt.% and 25 wt.% during the contacting.

**25.** The method as in claim 22 or 23 wherein the liquid mass transfer agent is water and the water total is maintained at between about 5 wt.% and 15 wt.% during the contacting.

**26.** The method as in claim 22 or 23 wherein the contacting is conducted in an extruder.

**27.** The method of claim 22 wherein the solid neutralized intermediate has the structure

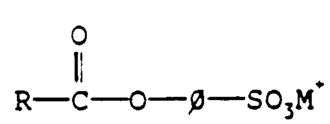

and R is $R^1$, $-OR^2$, or

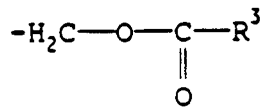

where $R^1$, $^2$, or $^3$ is a hydrocarbon radical containing up to about 30 carbon atoms and is selected from the group consisting of alkyl, alkenyl, cycloalkyl, aryl, aralkyl, and alkaryl, and $M^+$ is an alkali metal salt.

**28.** The method of claim 22 wherein the solid neutralized intermediate has the structure

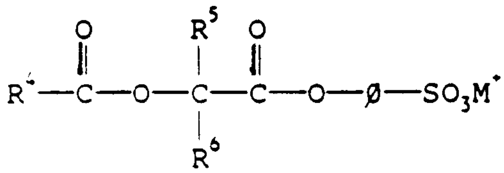

wherein $R^4$ is a $C_{1-20}$ linear or branched alkyl, alkoxylated alkyl, cycloalkyl, aryl, substituted aryl; $R^5$ and $R^6$ are independently H, $C_{1-20}$ alkyl, aryl, $C_{1-20}$ alkylaryl, substituted aryl, and $NR_3{}^{a+}$, wherein $R^a$ is $C_{1-30}$ alkyl, and $M^+$ is an alkali metal salt.

**29.** The method of claim 22 wherein the solid neutralized intermediate has the structure

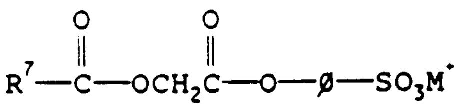

and $R^7$ is a branched or unbranched alkyl of 5-9 carbons and $M^+$ is an alkali metal salt.

**Patentansprüche**

1. Verfahren zur Herstellung von Reinigungs-, Bleich- oder Stoffbehandlungsadditiven, dadurch **gekennzeichnet,** daß ein Ausgangszwischenprodukt mit einem acyloxysubstituierten Benzolring ausgewählt wird;
   der Benzolring des Ausgangszwischenprodukts sulfoniert wird, wobei das Sulfonieren (a) die Behandlung des Ausgangszwischenprodukts mit einer Schwefeltrioxidquelle, wobei die Schwefeltrioxidquelle mindestens etwa einen 30%igen molaren Überschuß an Schwefeltrioxid, als er stöchiometrisch für die Sulfonierung des Benzolrings zu einem sulfonierten Zwischenprodukt erforderlich ist, liefert, und dann (b) die irreversible Umsetzung des Überschusses an Schwefeltrioxid mit mindestens einem Abschreckmittel in Anwesenheit des sulfonierten Zwischenprodukts umfaßt.

2. Verfahren nach Anspruch 1, dadurch **gekennzeichnet**, daß das gesamte Abschreckmittel in einer Menge von etwa mindestens 0,6:1, bezogen auf den molaren Überschuß an Schwefeltrioxid, zugegeben wird.

3. Verfahren nach Anspruch 1, dadurch **gekennzeichnet,** daß es weiter die Neutralisation des sulfonierten Zwischenprodukts unter Bildung einer Persäurevorstufe umfaßt.

4. Verfahren nach Anspruch 1, dadurch **gekennzeichnet,** daß mindestens ein Abschreckmittel mindestens einen aromatischen Ring hat oder eine Carbonsäure oder ein Keton ist.

5. Verfahren nach Anspruch 4, dadurch **gekennzeichnet,** daß der aromatische Ring unsubstituiert oder mit mindestens einer aktivierenden Gruppe substituiert ist.

6. Verfahren nach Anspruch 5, dadurch **gekennzeichnet,** daß die aktivierende Gruppe ausgewählt wird aus der Gruppe, die besteht aus $-NH_2$, -OH, $-OCH_3$, $-NHCOCH_3$, $-C_6H_5$, $-CH_3$ oder $C_nH_{2n+1}$, worin n eine ganze Zahl bis zu etwa 20 bedeutet.

7. Verfahren nach Anspruch 1, dadurch **gekennzeichnet,** daß mindestens ein Abschreckmittel Toluol, ein lineares Alkylbenzol, Xylol, eine Carbonsäure oder ein Keton ist und, wenn mehr als ein Abschreckmittel verwendet wird, dann die Abschreckmittel gemeinsam oder aufeinanderfolgend zugegeben werden.

8. Verfahren nach Anspruch 1, dadurch **gekennzeichnet,** daß das Ausgangszwischenprodukt die Struktur

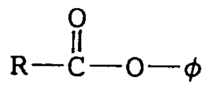

bedeutet, worin R, $R^1$, $-OR^2$ oder

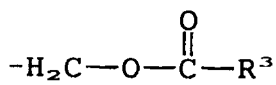

bedeutet, worin $R^1$, $R^2$ oder $R^3$ eine Kohlenwasserstoffgruppe, welche bis zu 30 Kohlenstoffatome enthält, bedeuten und ausgewählt werden aus der Gruppe, die besteht aus Alkyl, Alkenyl, Cvcloalkyl, Aryl, Aralkyl und Alkaryl.

9. Verfahren nach Anspruch 1, dadurch **gekennzeichnet,** daß das Ausgangszwischenprodukt die Struktur

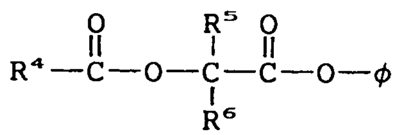

besitzt, worin $R^4$ $C_{1-20}$-lineares oder verzweigtes Alkyl, ethoxyliertes Alkyl, Cycloalkyl, Aryl, substituiertes Aryl bedeutet; $R^5$ und $R^6$ unabhängig H, $C_{1-20}$-Alkyl, Aryl, $C_{1-20}$-Alkylaryl, substituiertes Aryl und $NR_3{}^{a+}$ bedeuten, worin $R^a$ $C_{1\ 30}$-Alkyl bedeutet.

10. Verfahren nach Anspruch 1, dadurch **gekennzeichnet,** daß das Ausgangszwischenprodukt die Struktur

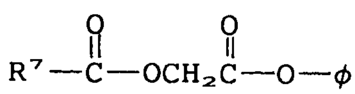

besitzt und $R^7$ verzweigtes oder unverzweigtes Alkyl mit etwa 5 bis etwa 9 Kohlenstoffen bedeutet.

11. Verfahren nach Anspruch 9 oder 10, dadurch **gekennzeichnet,** daß mindestens ein Abschreckmittel, das zugegeben wird, Xylol, eine Carbonsäure, ein Keton, Toluol oder ein lineares Alkylbenzol ist.

12. Verfahren nach Anspruch 9 oder 10, dadurch **gekennzeichnet,** daß das gesamte Abschreckmittel in einer Menge vorhanden ist, die ungefähr gleich ist dem molaren Überschuß an Schwefeltrioxid.

13. Verfahren nach Anspruch 1, dadurch **gekennzeichnet,** daß die Behandlung der Stufe (a) die Überprüfung entweder einer Menge eines ersten Reaktions-Nebenprodukts oder einer Menge des sulfonierten Zwischenprodukts, welches vor der Komplexbildung der Stufe (b) gebildet wird, und die Zugabe von mindestens einem Abschreckmittel, wenn die Menge, die geprüft wird, einen ausgewählten Wert erreicht, umfaßt.

14. Verfahren nach Anspruch 3, dadurch **gekennzeichnet,** daß die irreversible Reaktion der Stufe (b) die Überprüfung entweder einer Menge eines zweiten Reaktionsnebenprodukts oder einer Menge eines sulfonierten Zwischenprodukts, das vor der Zugabe des Abschreckmittels gebildet wurde, und die Neutralisation, wenn die geprüfte Menge einen ausgewählten Wert erreicht, umfaßt.

15. Verfahren nach Anspruch 3, dadurch **gekennzeichnet,** daß die Neutralisation die Behandlung des sulfonierten Zwischenprodukts mit einem Neutralisationsmittel, um die Sulfonsäure zu neutralisieren und eine Persäurevorstufe zu bilden, und die Ausfällung im wesentlichen der gesamten Persäurevorstufe als Salz in fester Phase umfaßt.

16. Verfahren nach Anspruch 15, dadurch **gekennzeichnet,** daß das Neutralisationsmittel ein Alkalimetallcarbonat, -bicarbonat oder ein Gemisch davon ist.

17. Verfahren nach Anspruch 15, dadurch **gekennzeichnet,** daß das Neutralisationsmittel ein Gemisch aus Kalium- oder Natriumhydroxid und einem Alkalimetallcarbonat umfaßt.

18. Verfahren nach Anspruch 15, dadurch **gekennzeichnet,** daß das Neutralisationsmittel ein Gemisch aus Alkalimetallpolyacrylat und Alkalimetallcarbonat umfaßt.

19. Verfahren nach Anspruch 15, dadurch **gekennzeichnet,** daß das Neutralisationsmittel ein Gemisch aus Alkalimetallpolyacrylat, Alkalimetallhydroxid und/oder Alkalimetallcarbonat umfaßt.

20. Verfahren nach Anspruch 15, dadurch **gekennzeichnet,** daß die Behandlung in Anwesenheit einer ausgewählten Menge an Wasser erfolgt.

21. Verfahren nach Anspruch 20, dadurch **gekennzeichnet,** daß das Abschreckmittel Toluol, Carbonsäure oder lineares Alkylbenzol, ein Gemisch aus Toluol und linearem Alkylbenzol oder ein Gemisch aus Toluol und Carbonsäure ist und daß das entstehende sulfonierte Zwischenprodukt und das Nebenprodukt beide neutralisiert werden.

22. Verfahren zur Herstellung von Persäurevorstufen in hoher Ausbeute durch Neutralisation sulfonierter Arylester, dadurch **gekennzeichnet**, daß eine Neutralisationszubereitung zur Verfügung gestellt wird, wobei die Komponenten davon im wesentlichen aus der zu neutralisierenden Zwischenproduktverbindung, einem Neutralisationsmittel und einem flüssigen Massenübertragungsmittel bestehen, die Zwischenproduktverbindung eine Arylestergruppierung mit einem Sulfonsäuresubstituenten an dem aromatischen Ring hat, das Neutralisationsmittel eine Quelle für Kationen liefert, welche mit den Wasserstoffionen des Sulfonsäuresubstituenten für dessen Neutralisation austauschbar sind und das flüssige Massenübertragungmittel den Austausch der Kationen und der Wasserstoffionen in flüssigem Medium erlaubt, wobei bei einem solchen Austausch eine Persäurevorstufe als neutralisiertes Zwischenprodukt gebildet wird, das Neutralisationsmittel und das flüssige Massenübertragungsmittel der Zubereitung so ausgewählt werden, daß die Bildung eines neutralisierten Zwischenprodukts als Feststoff innerhalb der Neutralisationszubereitung möglich wird, wenn die Komponenten davon miteinander in innigen Kontakt gebracht werden, und die Komponenten der Zubereitung innigst in Kontakt gebracht werden, wobei ein neutralisiertes Zwischenprodukt als Feststoff innerhalb der Neutralisationszubereitung während der Behandlung gebildet wird, wobei das feste neutralisierte Zwischenprodukt im wesentlichen gegenüber der Hydrolyse durch die Neutralisationszubereitung resistent ist.

23. Verfahren nach Anspruch 22, dadurch **gekennzeichnet,** daß das Neutralisationsmittel ausgewählt wird aus der Gruppe, die besteht aus Alkalimetallhydroxid und Alkalimetallpolyacrylat, Alkalimetallcarbonat, Alkalimetallbicarbonat und deren Gemischen.

24. Verfahren nach Anspruch 22 oder 23, dadurch **gekennzeichnet,** daß das flüssige Massenübertragungsmittel Wasser ist und daß die Gesamtmenge an Wasser zwischen etwa 5 und 25 Gew.-% während der Behandlung gehalten wird.

25. Verfahren nach Anspruch 22 oder 23, dadurch **gekennzeichnet,** daß das flüssige Massenübertragungsmittel Wasser ist und daß die Gesamtmenge an Wasser zwischen etwa 5 und 15 Gew.-% während der Behandlung gehalten wird.

26. Verfahren nach Anspruch 22 oder 23, dadurch **gekennzeichnet,** daß die Behandlung in einem Extruder erfolgt.

27. Verfahren nach Anspruch 22, dadurch **gekennzeichnet,** daß das feste neutralisierte Zwischenprodukt die Struktur

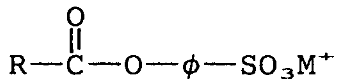

besitzt und R, $R^1$, $-OR^2$ oder

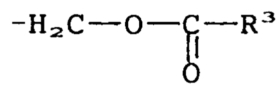

bedeutet, worin $R^1$, $R^2$ oder $R^3$ eine Kohlenwasserstoffgruppe, welche bis zu etwa 30 Kohlenstoffatome enthält, bedeutet und ausgewählt wird aus der Gruppe, die besteht aus Alkyl, Alkenyl, Cycloalkyl, Aryl, Aralkyl und Alkaryl, und $M^+$ ein Alkalimetallsalz bedeutet.

28. Verfahren nach Anspruch 22, dadurch **gekennzeichnet**, daß das feste neutralisierte Zwischenprodukt die Struktur

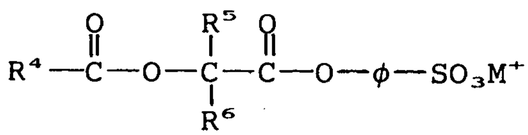

besitzt, worin $R^4$ $C_{1-20}$-lineares oder verzweigtes Alkyl, alkoxyliertes Alkyl, Cycloalkyl, Aryl, substituiertes Aryl bedeutet; $R^5$ und $R^6$ unabhängig H, $C_{1-20}$-Alkyl, Aryl, $C_{1-20}$-Alkylaryl, substituiertes Aryl und $NR_3{}^{a+}$ bedeuten, worin $R^a$ $C_{1\ 30}$-Alkyl bedeutet und $M^+$ ein Alkalimetallsalz bedeutet.

29. Verfahren nach Anspruch 22, dadurch **gekennzeichnet**, daß das feste neutralisierte Zwischenprodukt die Struktur

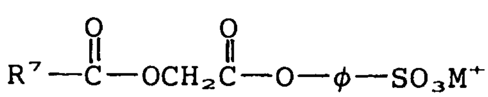

besitzt und $R^7$ verzweigtes oder unverzweigtes Alkyl mit 5 bis 9 Kohlenstoffen bedeutet und $M^+$ ein Alkalimetallsalz bedeutet.

## Revendications

1. Procédé pour la production d'additifs de nettoyage, de blanchiment ou de traitement des tissus, comprenant les opérations consistant à :
sélectionner un agent intermédiaire initial ayant un cycle benzène substitué par acyloxy ;
sulfoner le cycle benzène de l'agent intermédiaire initial, la sulfonation comprenant les étapes consistant à (a) mettre en contact l'agent intermédiaire initial avec une source de trioxyde de soufre, la source de trioxyde de soufre fournissant au moins environ un excédent de 30 % molaire de trioxyde de soufre que ce qui est requis stoëchiométriquement pour la sulfonation du cycle benzène pour former un agent intermédiaire sulfoné et ensuite (b) à faire irréversiblement réagir l'excédent de trioxyde de soufre avec au moins un agent d'extinction en présence de l'agent intermédiaire sulfoné.

2. Procédé selon la revendication 1, dans lequel l'agent d'extinction total est additionné dans une quantité d'environ au moins 0,6:1 par rapport à l'excédent molaire du trioxyde de soufre.

3. Procédé selon la revendication 1, comprenant de plus :
la neutralisation de l'agent intermédiaire sulfoné pour former un précurseur peracide.

4. Procédé selon la revendication 1, dans lequel au moins un agent d'extinction possède au moins un cycle aromatique ou il s'agit d'un acide carboxylique ou d'une cétone.

5. Procédé selon la revendication 4, dans lequel le cycle aromatique est non substitué ou il est substitué par au moins un groupe d'activation.

6. Procédé selon la revendication 5, dans lequel le groupe d'activation est choisi dans le groupe constitué par $-NH_2$, $-OH$, $-OCH_3$, $-NHCOCH_3$, $-C_6H_5$, $-CH_3$ ou $C_nH_{2n+1}$, dans lequel n est un nombre entier jusqu'à environ 20.

7. Procédé selon la revendication 1, dans lequel au moins un agent d'extinction est le toluène, un alkyle linéaire, benzène, xylène, un acide carboxylique ou une cétone et lorsque l'on a plus d'un agent d'extinction, ces agents d'extinction sont alors ajoutés ensemble ou séquentiellement.

8. Procédé selon la revendication 1, dans lequel l'agent intermédiaire initial a la structure

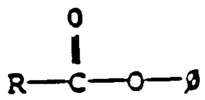

R est $R^1$, $-OR^2$, ou

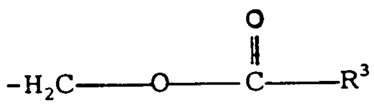

où $R^1$, $R^2$ ou $R^3$ est un radical d'hydrocarbure contenant jusqu'à environ 30 atomes de carbone et il est choisi dans le groupe constitué par alkyle, alcényle, cycloalkyle, aryle, aralkyle et alkaryle.

9. Procédé selon la revendication 1, dans lequel l'agent intermédiaire initial a la structure

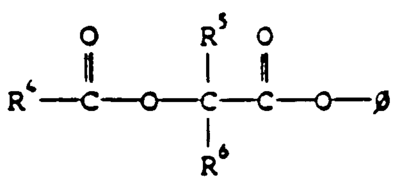

dans laquelle $R^4$ est un alkyle linéaire ou ramifié $C_{1-20}$, alkyléthoxylé, cycloalkyle, aryle, aryle substitué ; $R^5$ et $R^6$ sont indépendamment H, alkyle $C_{1-20}$, aryle, alkylaryle $C_{1-20}$, aryle substitué, et $NR_3^{a+}$ , où $R^a$ est alkyle $C_{1-30}$.

10. Procédé selon la revendication 1, dans lequel l'agent intermédiaire initial a la structure

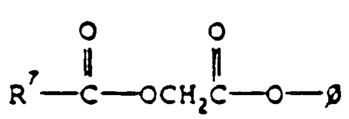

et $R^7$ est un alkyle ramifié ou non ramifié avec environ 5 jusqu'à environ 9 atomes de carbone.

11. Procédé selon la revendication 9 ou 10, dans lequel au moins un agent d'extinction ajouté est le xylène, un acide carboxylique, une cétone, un toluène ou un alkylbenzène linéaire.

**12.** Procédé selon la revendication 9 ou 10, dans lequel l'agent d'extinction total est présent dans une quantité environ égale à l'excédent molaire du trioxyde de soufre.

**13.** Procédé selon la revendication 1, dans lequel la mise en contact de l'étape (a) comprend le contrôle soit d'une quantité d'un premier sous-produit réactionnel soit d'une quantité de produit intermédiaire sulfoné qui est transformé avant le complexage de l'étape (b), l'addition d'au moins un agent d'extinction lorsque la quantité contrôlée atteint une valeur sélectionnée.

**14.** Procédé selon la revendication 3, dans lequel la réaction irréversible de l'étape (b) comprend le contrôle soit d'une quantité d'un second sous-produit réactionnel soit d'une quantité de produit intermédiaire sulfoné formé avant addition de l'agent d'extinction, et neutralisation lorsque la quantité à contrôler atteint une valeur sélectionnée.

**15.** Procédé selon la revendication 3, dans lequel la neutralisation comprend la mise en contact du produit intermédiaire sulfoné avec un agent neutralisant pour neutraliser l'acide sulfonique et pour former un précurseur peracide, et la précipitation de sensiblement tout le précurseur peracide sous forme de sel en phase solide.

**16.** Procédé selon la revendication 15, dans lequel l'agent neutralisant est un carbonate des métaux alcalins, un bicarbonate ou son mélange.

**17.** Procédé selon la revendication 15, dans lequel l'agent neutralisant comprend un mélange d'hydroxyde de sodium ou de potassium et un carbonate des métaux alcalins.

**18.** Procédé selon la revendication 15, dans lequel l'agent neutralisant comprend un mélange de polyacrylate des métaux alcalins et de carbonate des métaux alcalins.

**19.** Procédé selon la revendication 15, dans lequel l'agent neutralisant comprend un mélange de polyacrylate des métaux alcalins, d'hydroxyde des métaux alcalins et/ou de carbonate des métaux alcalins.

**20.** Procédé selon la revendication 15, dans lequel la mise en contact s'effectue en présence d'une quantité sélectionnée d'eau.

**21.** Procédé selon la revendication 20, dans lequel l'agent d'extinction est le toluène, l'acide carboxylique, ou un alkylbenzène linéaire, un mélange de toluène et un alkylbenzène linéaire, ou un mélange de toluène et d'acide carboxylique, et le produit intermédiaire sulfoné résultant et le sous-produit sont tous deux neutralisés.

**22.** Procédé pour la préparation de précurseurs peracides en rendement élevé en neutralisant les arylesters sulfonés, comprenant :

la mise en place d'une formulation neutralisante avec ces composants, constituée essentiellement par un composé intermédiaire à neutraliser, un agent de neutralisation, et un agent de transfert de masse liquide, le composé intermédiaire possédant une partie d'arylester avec un substituant d'acide sulfonique sur le cycle aromatique, l'agent de neutralisation fournissant une source de cations échangeables avec les ions hydrogène du substituant d'acide sulfonique pour sa neutralisation, et l'agent de transfert de masse liquide permettant l'échange des cations et des ions hydrogène dans un milieu liquide, cet échange formant un précurseur peracide sous forme de produit intermédiaire neutralisé, l'agent de neutralisation et l'agent de transfert de masse liquide de la formulation étant choisis pour permettre la formation de produit intermédiaire neutralisé sous la forme solide dans la formulation de neutralisation lorsque ces composants sont mis en contact intime ; et

mise en contact intime de ces composants de formulation pour former un produit intermédiaire neutralisé sous la forme solide à l'intérieur de la formulation de neutralisation pendant la mise en contact, le produit intermédiaire neutralisé solide étant sensiblement résistant à l'hydrolyse par la formulation de neutralisation.

**23.** Procédé selon la revendication 22, dans lequel l'agent de neutralisation est choisi dans le groupe constitué par un polyacrylate des métaux alcalins et un hydroxyde des métaux alcalins, un carbonate des métaux alcalins, un bicarbonate des métaux alcalins, et leurs mélanges.

24. Procédé selon la revendication 22 ou 23, dans lequel l'agent de transfert de masse liquide est de l'eau et le total d'eau est maintenu à environ 5 % en poids et 25 % en poids pendant la mise en contact.

25. Procédé selon la revendication 22 ou 23, dans lequel l'agent de transfert de masse liquide est de l'eau et le total de l'eau est maintenu entre environ 5 % en poids et 15 % en poids pendant la mise en contact.

26. Procédé selon la revendication 22 ou 23, dans lequel la mise en contact est conduite dans une extrudeuse.

27. Procédé selon la revendication 22, dans lequel l'agent intermédiaire neutralisé solide a la structure

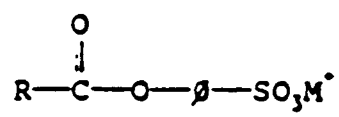

et R est $R^1$, $-OR^2$, ou où $R^1$, $^2$ ou $^3$ est un

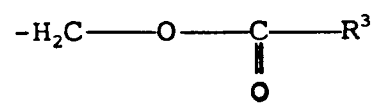

radical d'hydrocarbure contenant jusqu'à environ 30 atomes de carbone et il est choisi dans le groupe constitué par alkyle, alcényle, cycloalkyle, aryle, aralkyle et alkaryle, et $M^+$ est un sel des métaux alcalins.

28. Procédé selon la revendication 22, dans lequel le produit intermédiaire neutralisé solide a la structure

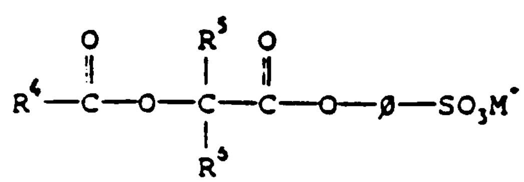

dans laquelle $R^4$ est un alkyle linéaire ou ramifié $C_{1-20}$, un alkyle alcoxylé, un cycloalkyle, un aryle, un aryle substitué ; $R^5$ et $R^6$ sont indépendamment H, alkyle $C_{1-20}$, aryle, alkylaryle $C_{1-20}$, aryle substitué, et $NR_3^{a+}$ , dans lequel $R^a$ est alkyle $C_{1-30}$ et $M^+$ est un sel des métaux alcalins.

29. Procédé selon la revendication 22, dans lequel l'agent intermédiaire neutralisé solide a la structure

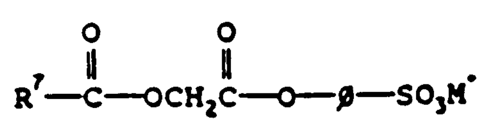

et $R^7$ est un alkyle ramifié ou non ramifié de 5-9 atomes de carbone et $M^+$ est un sel des métaux alcalins.

Fig. 1

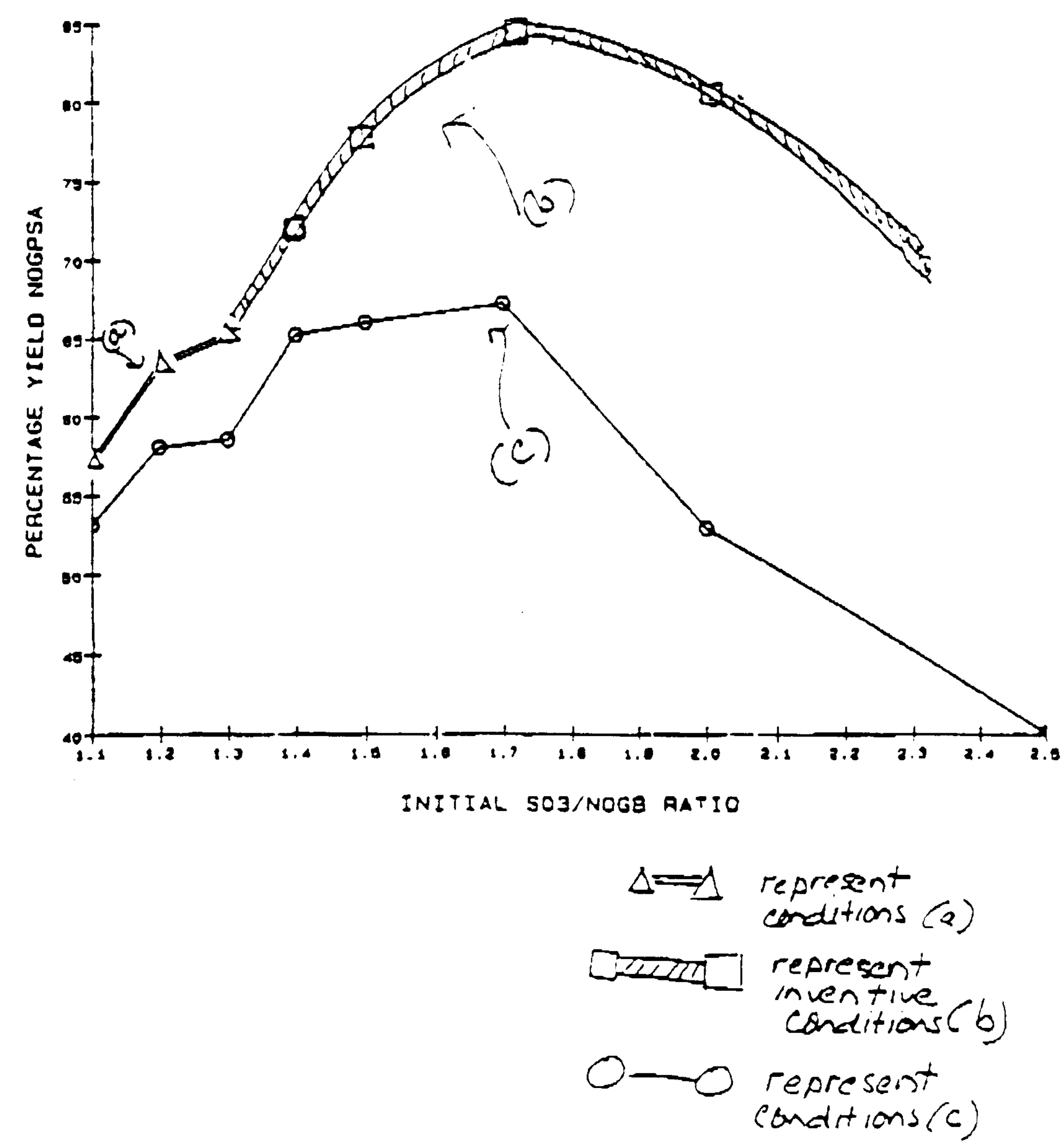
MOLAR YIELD
PERCENTAGE YIELD NOGPSA
85
80
75
70
65
60
55
50
45
40
1.1
1.2
1.3
1.4
1.5
1.6
1.7
1.8
1.9
2.0
2.1
2.2
2.3
2.4
2.5
INITIAL SO3/NOGB RATIO
(a)
(b)
(c)
represent conditions (a)
represent inventive conditions (b)
represent conditions (c)

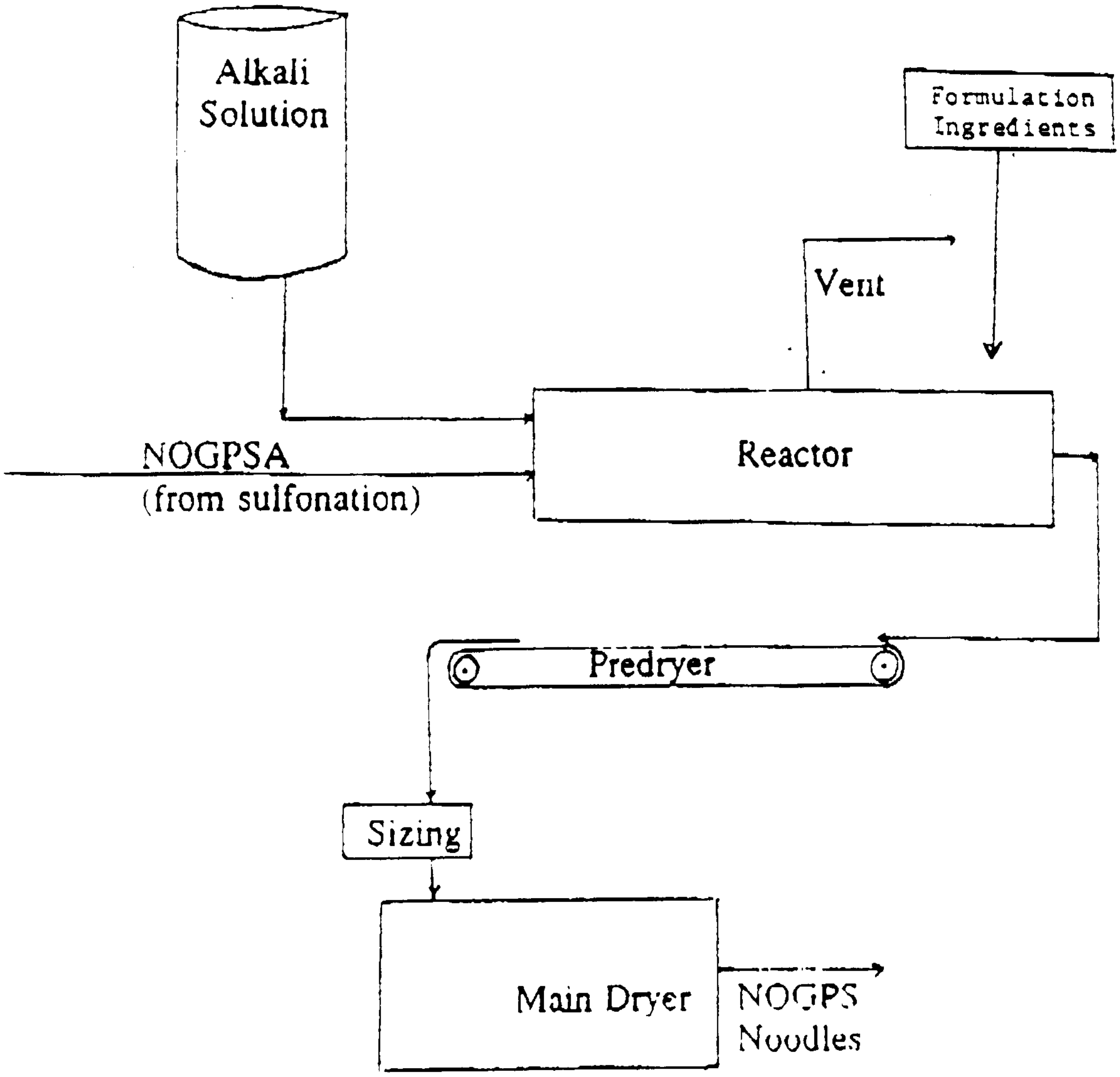
Alkali Solution
Formulation Ingredients
Vent
Reactor
NOGPSA
(from sulfonation)
Predryer
Sizing
Main Dryer
NOGPS Noodles

Fig. 2